(19) Europäisches Patentamt
European Patent Office
Office européen des brevets

(11) **EP 2 381 796 B1**

(12) **EUROPEAN PATENT SPECIFICATION**

(45) Date of publication and mention
of the grant of the patent:
**20.02.2019 Bulletin 2019/08**

(21) Application number: **09797238.4**

(22) Date of filing: **29.12.2009**

(51) Int Cl.:
*A61P 3/04* (2006.01)          *A23K 20/121* (2016.01)
*A23K 50/40* (2016.01)

(86) International application number:
**PCT/US2009/069654**

(87) International publication number:
**WO 2010/078301 (08.07.2010 Gazette 2010/27)**

(54) **COMPOSITIONS AND METHODS FOR TREATING AND PREVENTING WEIGHT-RELATED DISORDERS IN COMPANION ANIMALS**

ZUSAMMENSETZUNGEN UND VERFAHREN FÜR DIE BEHANDLUNG UND VORBEUGUNG VON GEWICHTSPROBLEMEN BEI HEIMTIEREN

COMPOSITIONS ET PROCÉDÉS DE TRAITEMENT ET DE PRÉVENTION DE TROUBLES LIÉS AU POIDS CHEZ DES ANIMAUX DE COMPAGNIE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **30.12.2008 US 141354 P**

(43) Date of publication of application:
**02.11.2011 Bulletin 2011/44**

(73) Proprietor: **Hill's Pet Nutrition, Inc.
Topeka, KS 66603 (US)**

(72) Inventor: **FRANTZ, Nolan Zebulon
Andover
07821 New Jersey (US)**

(74) Representative: **Wichmann, Hendrik et al
Wuesthoff & Wuesthoff
Patentanwälte PartG mbB
Schweigerstraße 2
81541 München (DE)**

(56) References cited:
**WO-A1-2007/063095          DE-A1- 19 818 563
JP-A- 2007 308 468          US-A1- 2002 006 907
US-A1- 2003 224 061**

• **DATABASE WPI Week 200659 Thomson Scientific, London, GB; AN 2006-573787 XP002579928 & JP 2006 219467 A (ORIZA YUKA KK) 24 August 2006 (2006-08-24)**
• **Min-Seon Kim ET AL: "Anti-obesity effects of [alpha]-lipoic acid mediated by suppression of hypothalamic AMP-activated protein kinase", Nature Medicine, vol. 10, no. 7, 1 July 2004 (2004-07-01), pages 727-733, XP55375693, ISSN: 1078-8956, DOI: 10.1038/nm1061**

EP 2 381 796 B1

**Description**

**FIELD OF THE INVENTION**

The invention encompasses a composition comprising 100 ppm to 200 ppm alpha-lipoic acid for use in inducing loss of body fat or increasing the percentage of muscle mass in the treatment of a weight-related disorder in a companion animal in need thereof, which comprises feeding the companion animal said composition, wherein the companion animal is a dog.

**BACKGROUND OF THE INVENTION**

An important indicator of animal health is the body composition of the animal. An unhealthy diet and/or an unhealthy lifestyle can result in the animal having an unhealthy proportion of body fat, particularly in relation to lean muscle in the body. It is thought that a body fat amount in excess of 30% by weight indicates that the animal is unhealthy, particularly if the amount of body fat is in excess of 35% by weight.
The invention generally relates to pet food compositions for dogs, which have increased therapeutic and prophylactic efficacy over currently marketed companion food products for maintaining or promoting a healthy body composition.
WO 2007/063095 discloses the use of zinc salts of lipoic acid in the treatment of obesity.
US 2003/0224061 discloses oral compositions with insulin-like activities.
DE 19818563 discloses compositions comprising lipoic acid for use in the reduction of appetite.
JP 2007-308468 discloses a basal metabolism increasing agent.
Database WPI Week 200659 Thomson Scientific, London, GB; AN 2006-573787 % JP 2006 219467 A (ORIZA YUKA KK) 24 August 2006 discloses a composition comprising alpha-lipoic acid as an antilipemic or anorectic.
US 2002/006907 discloses alpha-lipoic acid compositions for increasing muscle size in humans.
Kim et al.. Nature Medicine, 10(7), pages 727-733 (2004) discloses weight loss and decreased visceral fat mass in rats after administration of compositions comprising 0.25% to 1% of lipoic acid.

SUMMARY OF THE INVENTION

The inventors have developed food compositions for treating weight-related disorders in animals, as defined in the claims. In one aspect, the present invention provides a composition comprising 100 ppm to 200 ppmalpha-lipoic acid for use in inducing loss of body fat or increasing the percentage of muscle mass in the treatment of a weight-related disorder in a companion animal in need thereof, which comprises feeding the companion animal said composition, wherein the companion animal is a dog. In embodiments, the composition is to be administered for at least 15 days, for at least 30 days, or for at least 45 days. In embodiments, the composition is to be administered daily.
Described herein are methods for maintaining or promoting a healthy body composition, for example, loss of body weight or body fat, increased percentage of muscle mass in a companion animal, which includes feeding the animal a composition including lipoic acid or a salt thereof in an amount effective to promote or maintain healthy body composition.

DETAILED DESCRIPTION

General Description

The present disclosure encompasses food compositions including an effective amount of lipoic acid or salt thereof to prevent or treat disorders in a companion animal in need thereof.
One embodiment encompasses methods for inducing loss of body fat in a companion animal, which includes feeding the animal a composition of the invention, which includes alpha-lipoic acid in an amount effective to induce loss of body fat, wherein the companion animal is a dog. The effective amount is 100 ppm to 200 ppm.
In certain embodiments, the composition further comprises a protein, fat, carbohydrate, fiber, and combinations thereof.
In certain embodiments, the composition is a dog food.
In certain embodiments, the composition is a food, a nutritional diet, a supplement, an animal treat, or a toy.
In certain embodiments, the composition is in the form of a moist food.
In certain embodiments, the composition is in the form of a dry food.
Another embodiment encompasses methods for increasing the percentage of muscle mass in a companion animal, which included feeding the animal a composition of the invention, which includes alpha-lipoic acid in an amount effective to increase the percentage of muscle mass, wherein the companion animal is a dog. The effective amount is 100 ppm to 200 ppm.

In certain embodiments, the composition further comprises a protein, fat, carbohydrate, fiber, and combinations thereof.

In certain embodiments, the composition is a dog food.

In certain embodiments, the composition is a food, a nutritional diet, a supplement, an animal treat, or a toy.

In certain embodiments, the composition is in the form of a moist food.

In certain embodiments, the composition is in the form of a dry food.

The term "dog" includes those dogs, which are companion animals such as *Canis familiaris,* working dogs and the like. The term dog is synonymous with the term canine.

All percentages expressed herein are by weight of the composition on dry matter basis unless specifically stated otherwise.

Compositions of the Invention

One aspect of the invention provides a composition for use according to claim 1.

As used herein, the term "lipoic acid" includes alpha-lipoic acid and a racemic mixture of alpha-lipoic acid. In various embodiments, the lipoic acid can be administered in a composition comprising a wet or dry food composition, which may be in the form of a moist food, dry food, supplement or treat. The lipoic acid may be incorporated therein or on the surface of any food composition, such as, by spraying or precipitation thereon or may be added to the diet by way of snack, supplement, treat or in the liquid portion of the diet such as water or another fluid. The lipoic acid may be administered as a powder, solid or as a liquid including a gel. An important aspect is that the animal be provided an effective amount of the lipoic acid to provide a positive effect. Typically, the source of lipoic acid is present in the composition in an amount of up to an amount which remains non-toxic to the animal.

The amount of alpha-lipoic acid in the compositions for use of the present invention is 100 ppm to 200 ppm. In a reference example, the quantity of alpha-lipoic acid can vary from at least about 25 ppm, about 50 ppm, about 200 ppm, about 300 ppm, about 500 ppm, about 700 ppm, about 900 ppm, about 1100 ppm, about 1200 ppm, about 1400 ppm, about 1600 ppm, about 1800 ppm, about 2000 ppm, about 2200 ppm, about 2400 ppm, about 2600 ppm, about 2800 ppm, about 3000 ppm, or about 3500 ppm. In various embodiments, the range of lipoic acid that can be administered dogs is about 150 ppm to about 4500 ppm. In certain illustrative embodiments, quantities can vary from about 100 ppm to an amount which remains nontoxic to the pet.

In various embodiments, a food composition comprising lipoic acid provides a substantially nutritionally complete diet for the intended recipient animal. A "nutritionally complete diet" is a diet that includes sufficient nutrients for maintenance of normal health of a healthy animal on the diet.

The lipoic acid is present at a concentration that is not deleterious to the intended animal's health. Thus, for example, the lipoic acid or salt thereof is present at a concentration that does not cause undesirable or toxic effects.

The composition can be a liquid or a solid food. When the composition is a liquid, the lipoic acid can be admixed with other components. Where the composition is solid, the lipoic acid may be coated on the composition, incorporated into the composition, or both.

In various embodiments, the lipoic acid may be added to the animal's food. In various embodiments, the lipoic acid may be added to the animal's food by a compounder or manufacturer at a site or by an animal's caregiver prior to feeding the animal. In various embodiments, the lipoic acid may be added during the processing of an animal's food, such as during and/or after mixing of other components of the composition that is then packaged and made available to consumers. Such processing may include extrusion, canning, baking, and the like or any other method or process of producing pet foods that is known in the art. In various embodiments, the lipoic acid may be contributed by a natural source like an animal or plant component, or the lipoic acid may be contributed by a synthetically derived source, or the lipoic acid may be contributed by a mixture of natural and synthetic sources.

The compositions in addition to lipoic acid include at least one component suitable for consumption by a companion animal including, but not limited to, fats, carbohydrates, proteins, fibers, nutritional balancing agents such as vitamins, minerals, and trace elements, and mixtures thereof. One of ordinary skill in the art can select the amount and type of food ingredients for a typical food based upon the dietary requirements of the animal, for example, the animal's species, age, size, weight, health, and function.

The food ingredient part of the food composition can include up to about 100% of any particular food ingredient or can include a mixture of food ingredients in various proportions. In certain embodiments, the food composition includes a combination of food ingredients in amounts of about 0 wt. % to about 50 wt. % fat, about 0 wt. % to about 75 wt. % carbohydrate, about 0 wt. % to about 95 wt. % protein, about 0 wt. % to about 40 wt. % dietary fiber, and about 0 wt. % to about 15 wt. % of one or more nutritional balancing agents.

In certain embodiments, the fat and carbohydrate food ingredient is obtained from a variety of sources such as animal fat, fish oil, vegetable oil, meat, meat by-products, grains, other animal or plant sources, and mixtures thereof.

Grains include wheat, corn, barley, and rice.

In certain embodiments, the protein food ingredient is obtained from a variety sources such as plants, animals, or both. Animal protein includes meat, meat by-products, dairy, and eggs. Meats include the flesh from poultry, fish, and animals such as cattle, swine, sheep, goats, and the like, meat by-products include lungs, kidneys, brain, livers, stomachs, and intestines. The protein food ingredient may also be free amino acids and/or peptides. Preferably, the protein food ingredient includes meat, a meat by-product, dairy products, or eggs.

In certain embodiments, the fiber food ingredient is obtained from a variety of sources such as vegetable fiber sources, for example, cellulose, beet pulp, peanut hulls, and soy fiber.

In certain embodiments, the nutritional balancing agents are obtained from a variety of sources known to skilled artisans, for example, vitamin and mineral supplements and food ingredients. Vitamins and minerals can be included in amounts required to avoid deficiency and maintain health. These amounts are readily available in the art. The National Research Council (NRC) provides recommended amounts of such nutrients for farm animals. See, e.g., Nutrient Requirements of Swine (10th Rev. Ed., Nat'l Academy Press, Wash. D.C., 1998), Nutrient Requirements of Poultry (9th Rev. Ed., Nat'l Academy Press, Wash. D.C., 1994), Nutrient Requirements of Horses (5th Rev. Ed., Nat'l Academy Press, Wash. D.C., 1989). The American Feed Control Officials (AAFCO) provides recommended amounts of such nutrients for dogs and cats. See American Feed Control Officials, Inc., Official publication, pp. 129-137 (2004). Vitamins generally useful as food additives include vitamin A, vitamin B1, vitamin B2, vitamin B6, vitamin B12, vitamin D, biotin, vitamin K, folic acid, inositol, niacin, and pantothenic acid. Minerals and trace elements useful as food additives include calcium, phosphorus, sodium, potassium, magnesium, copper, zinc, chloride, iron, selenium, iodine, and iron.

In certain embodiments, the food compositions may contain additional ingredients such as vitamins, minerals, fillers, palatability enhancers, binding agents, flavors, stabilizers, emulsifiers, sweeteners, colorants, buffers, salts, coatings, and the like known to skilled artisans. Stabilizers include substances that tend to increase the shelf life of the composition such as preservatives, synergists and sequestrants, packaging gases, stabilizers, emulsifiers, thickeners, gelling agents, and humectants. Examples of emulsifiers and/or thickening agents include gelatin, cellulose ethers, starch, starch esters, starch ethers, and modified starches. Specific amounts for each composition component, food ingredient, and other ingredients will depend on a variety of factors such as the particular components and ingredients included in the composition; the species of animal; the animal's age, body weight, general health, sex, and diet: the animal's consumption rate; the type of disease or condition being treated; and the like. Therefore, the component and ingredient amounts may vary widely and may deviate from the preferred proportions described herein.

In one illustrative embodiment, the composition may, for example, in addition to lipoic acid or a salt thereof also include at least one of the following:

    (a) about 0% to about 75% carbohydrate,
    (b) about 2% to about 50% fat,
    (c) about 0% to about 40% dietary fiber, and
    (d) about 0% to about 15% of one or more nutritional balancing agents.

The diet fed to the adult companion pet, for example, canine and feline is the standard normal diet fed to an animal of that age. Below is a typical diet for a canine of 1 to 6 years of age.

**Table 1: Illustrative Companion Animal Pet Food Composition**

| Ingredient | Target |
|---|---|
| Protein (% of dry matter) | 23 |
| Fat (% of dry matter) | 15 |
| Phosphorous (% of dry matter) | 0.6 |
| Sodium (% of dry matter) | 0.3 |

The compositions can contain additional ingredients intended to maintain or improve the health of the animal, for example, supplements, medications, herbs, holistic drugs and compositions, and the like.

The composition of the invention may include one or more additional ingredients to prevent or treat one or more diseases or conditions.

The component in the diet, which accomplishes this, is an antioxidant or mixture thereof. An antioxidant is a material that quenches a free radical. Examples of such materials include foods such as Ginkgo Biloba, citrus pulp, grape pomace, tomato pomace, carrot and spinach, all preferably dried as well as various other materials such as beta-carotene, selenium, coenzyme Q10 (ubiquinone), lutein, tocotrienols, soy isoflavones, S-adenosylmethionine, glutathione, taurine, N-acetylcysteine, Vitamin E, Vitamin C, alpha-lipoic acid, l-carnitine and the like. Vitamin E can be administered as a tocopherol or a mixture of tocopherols and various derivatives thereof such as esters like vitamin E acetate, succinate, palmitate, and the like. The alpha form is preferable but beta, gamma and delta forms can be included. The d form is preferable but racemic mixtures are acceptable. The forms and derivatives will function in a Vitamin E like activity after ingestion by the pet. Vitamin C can be administered in this diet as ascorbic acid and its various derivatives thereof such as calcium phosphate salts, cholesteryl salt, 2-monophosphate, and the like which will function in a vitamin C like activity after ingesting by the pet. They can be in any form such as liquid, semisolid, solid and heat stable form. Alpha-lipoic acid can be administered into the diet as alpha lipoic acid or as a lipoate derivative as in U.S. Pat. No. 5,621,117, racemic mixtures, salts, esters or amides thereof. L-carnitine can be administered in the diet and various derivatives of carnitine such as the salts such as the hydrochloride, fumarate and succinates, as well as acetylated carnitine, and the like can be used.

The quantities administered in the diet, all as wt % (dry matter basis) of the diet, are calculated as the active material, per se, that is measured as free material. The maximum amounts employed should not bring about toxicity. At least about 100 ppm or at least about 150 ppm of Vitamin E can be used. A preferred range of about 500 to about 1,000 ppm can be employed. Although not necessary, a maximum of about 2000 ppm or about 1500 ppm is generally not exceeded. With respect to Vitamin C at least about 50 ppm is used, desirably at least about 75 ppm and more desirably at least about 100 ppm. A non-toxic maximum can be employed. The quantity of alpha-lipoic acid can vary from 100 ppm to 200 ppm. For l-carnitine about 50 ppm, desirably about 200 ppm, more desirably about 300 ppm for canines are a useful minimum. A non-toxic maximum quantity can be employed, for example, less than about 5,000 ppm. For canines, lower quantities can be employed, for example, less than about 5,000 ppm. For canines, a preferred range is about 200 ppm to about 400 ppm. Beta-carotene at about 1-15 ppm can be employed. Selenium at about 0.1 up to about 5 ppm can be employed. Lutein at least about 5 ppm can be employed. Tocotrienols at least about 25 ppm can be employed. Coenzyme Q10 at least about 25 ppm can be employed. S-adenosylmethionine at least about 50 ppm can be employed. Taurine at least about 1000 ppm can be employed. Soy isoflavones at least about 25 ppm can be used. N-acetylcysteine at least about 50 ppm can be used. Glutathione at least about 50 ppm can be used. Gingko Biloba at least 50 ppm of extract can be used.

The following are raw ingredients that are high in ORAC (Oxygen radical absorbing capacity) content: Spinach pomace, Tomato pomace, Citrus Pulp, Grape Pomace, Carrot granules, Broccoli, Green tea, Ginkgo Biloba and Corn gluten meal.

When added to the diet as 1% inclusions (for a total of 5% substitution for a low ORAC ingredient such as corn) they increased the ORAC content of the overall diet and increased the ORAC content of the plasma of the animals which ate the diet containing these components. Preferably, any ingredient with an ORAC content >25 $\mu$mole of Trolox equivalents per gram of dry matter could be used if added at 1% combination with four other 1% ingredients for a total of 5% addition to the diet. In certain embodiments, the compositions further include an effective amount of at least one substance selected from the group consisting of glucosamine, chondroitin, chondroitin sulfate, methylsulfonylmethane ("MSM"), creatine, antioxidants, *Perna canaliculata,* omega-3 fatty acids, omega-6 fatty acids and mixtures thereof.

In various embodiments, a supplement including an effective amount of lipoic acid or a salt thereof further includes an effective amount of at least one substance including aspirin, anti-inflammatories such as ibuprofen, COX-2 inhibitors, and other medicinal and pharmaceutical compositions and combinations thereof. Supplements include, but are not limited to, a feed used with another feed to improve the nutritive balance or performance of the total. Supplements include compositions that are fed undiluted as a supplement to other feeds, offered free choice with other parts of an animal's ration that are separately available, or diluted and mixed with an animal's regular feed to produce a complete feed. The AAFCO, for example, provides a discussion relating to supplements in the American Feed Control Officials, Inc. Official Publication, p. 220 (2003). Supplements may be in various forms including, for example, powders, liquids, syrups, pills, and encapsulated compositions.

In certain embodiments, the composition can be a treat. Treats include compositions that are given to an animal to entice the animal to eat during a non-meal time, for example, dog bones for canines. Treats may be nutritional wherein the composition includes one or more nutrients and may have a food-like composition. Non-nutritional treats encompass any other treats that are non-toxic. The composition or components are coated onto the treat, incorporated into the treat, or both. Treats of the invention can be prepared by an extrusion or baking process similar to those used for dry food. Other processes also may be used to either coat the composition on the exterior of existing treat forms or inject the composition into an existing treat form.

In certain embodiments, the composition can be a toy. Toys include chewable toys such as artificial bones. The lipoic acid or a salt thereof can form a coating on the surface of the toy or on the surface of a component of the toy, be incorporated partially or fully throughout the toy, or both. In one embodiment, the lipoic acid or a salt thereof is orally accessible by the intended user. There are a wide range of suitable toys currently marketed, for example, U.S. Pat. No. 5,339,771, U.S. Pat. No. 5,419,283, and references disclosed therein. This invention provides both partially consumable toys, for example, toys including plastic components, and fully consumable toys, for example, rawhides and various artificial bones. The invention preferably provides toys for use by a dog or a cat.

Preparation of the Compositions for use according to the Invention

The compositions for use according to the invention may be prepared in a canned or wet form using conventional food preparation processes known to skilled artisans. Typically, ground animal proteinaceous tissues are mixed with the other ingredients such as fish oils, cereal grains, balancing ingredients, special purpose additives (e.g., vitamin and mineral mixtures, inorganic salts, cellulose and beet pulp, bulking agents, and the like) and water in amounts sufficient for processing. These ingredients are mixed in a vessel suitable for heating while blending the components. Heating of the mixture is effected using any suitable manner, for example, direct steam injection or using a vessel fitted with a heat exchanger. Following the addition of the last ingredient, the mixture is heated to a temperature of about 10 °C to about 100 °C (about 50 °F to about 212 °F). Temperatures outside this range are acceptable but may be commercially impractical without use of other processing aids. When heated to the appropriate temperature, the material will typically be in the form of a thick liquid. The thick liquid is filled into cans. A lid is applied, and the container is hermetically sealed. The sealed can is then placed into conventional equipment designed to sterilize the contents. Sterilization is usually accomplished by heating to temperatures of greater than about 110 °C (about 230 °F) for an appropriate time depending on the temperature used, the composition, and similar factors. The compositions of the present invention can be added to the food compositions before, during, or after preparation.

Food compositions may be prepared in a dry form using conventional processes known to skilled artisans. Typically, dry ingredients such as animal protein, plant protein, grains, and the like are ground and mixed together. Moist or liquid ingredients, including fats, oils, animal protein, water, and the like are then added to and mixed with the dry mix. The mixture is then processed into kibbles or similar dry pieces. Kibble is often formed using an extrusion process in which the mixture of dry and wet ingredients is subjected to mechanical work at a high pressure and temperature and forced through small openings and cut off into kibble by a rotating knife. The wet kibble is then dried and optionally coated with one or more topical coatings such as flavours, fats, oils, powders, and the like. Kibble also can be made from the dough using a baking process, rather than extrusion, wherein the dough is placed into a mold before dry-heat processing. The food compositions can be in the form of a treat using an extrusion or baking process similar to those described above for dry food or a toy such as those disclosed in U.S. Patent Nos. 5,339,771 and 5,419,283. The compositions of the present invention can be added to the food compositions before, during, or after preparation.

Methods of Treating or Preventing Disorders with Compositions of the Disclosure

The present disclosure also provides methods of treating or preventing certain disorders by administering a composition including an effective amount of alpha-lipoic acid to a companion animal in need thereof.

Adding quantities of an antioxidant or mixture thereof to the companion animal pet diet can maintain or promote a healthy body composition, for example, loss of weight or body fat, and increase percentage of muscle mass, in a pet, for example, an adult pet. The term, adult, is intended to mean, in general, a canine of at least 1 to 6 years. An aged dog is 7 years and above.

The inventors have succeeded in accomplishing maintaining or promoting a healthy body composition, for example, loss of weight or body fat, or increased percentage of muscle mass.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise.

Similarly, the words "include", "includes", and "including" are to be interpreted inclusively rather than exclusively. Unless defined otherwise, all technical and scientific terms and any acronyms used herein have the same meanings as commonly understood by one of ordinary skill in the art in the field of the invention. Although any compositions, methods, articles of manufacture, or other means or materials similar or equivalent to those described herein can be used in the practice of the present invention, the preferred compositions, methods, articles of manufacture, or other means or materials are described herein.

## EXAMPLES

This invention can be further illustrated by the following examples of preferred embodiments thereof, although it will be understood that these examples are included merely for purposes of illustration and are not intended to limit the scope of the invention unless otherwise specifically indicated.

EXAMPLE 1

30 adult, random source, dogs were utilized for this study. Dogs were at least 10 months of age, not pregnant, not lactating and of reasonable body weight prior to start of test. Animals were randomized into 5 groups for dietary treatment with 3 males and 3 females per each group.

All dogs were fed a control food (0 ppm dl-alpha-lipoic acid added) that met or exceeded all recommendations for nutrients as proposed by the American Association of Feed Control Officials (AAFCO 2000) during a 2 week prefeeding period (Table 2). Following the prefeeding period dogs were randomized into 5 treatment groups with one of the following dl-alpha lipoic acid target-inclusions (dry matter basis): 0 ppm, 150 ppm, 1500 ppm, 3000 ppm, 4500 ppm. In all diets, control and alpha lipoic acid, Vitamin E was added and was present at a level of 600-1000 International Units and Vitamin C was added at levels of 100-200 ppm.

Test foods were the sole source of nutrients except for water. Fresh water was provided *ad libitum*. After dogs were selected and initial body weights taken, a food dose was calculated for each dog based on the expected ME of the food. Initial food dose calculations were based on the maintenance energy requirement (MER) for the dog modified by a factor to account for normal activity as calculated by the following formula:

$$\text{MER(kcal/day)} = 1.6 \times \text{RER(Resting Energy Requirement)}$$

where:

$$\text{RER (kcal/day)} = 70 \times \text{body weight (kg)} 0.75$$

Dogs were weighed weekly and had food doses adjusted as needed in order to feed enough food to maintain their optimal body weight. Optimal body weight was determined to be 3 on a 5 point scale. If a dog did not maintain body weight within -10% of initial body weight, after adjustment of food dose, it was removed from the study. All measures of body weight and food intake were recorded.

Samples were ground and 0.100 +/-0.001 g of sample was extracted twice into 5.0 mL phosphate buffer (10 mM $Na_2HPO_4$, 2 mM ethylenediaminetetraacetic acid (EDTA), 0.9% NaCl, pH 7.4). 250 $\mu$L of extract was placed into a 5 mL glass centrifuge tube with a Teflon lined cap. 15 $\mu$L EDTA solution (100 mM EDTA, adjusted to pH 7.8 with about 1M NaOH) and 50 $\mu$L freshly prepared 5 mM dithioerythritol (DTE) were added. The solutions were vortexed and incubated at room temperature for 5 minutes. Then 10 $\mu$L of 1M $H_3PO_4$ and 2.0 mL diethyl ether were added. The tubes were capped, vortexed, and centrifuged at 1500 x g for 3 minutes at room temperature. The ether layer was transferred to a separate 5 mL glass centrifuge tube, while the aqueous layer was extracted twice more with 1.5 mL ether. All extractions from the same sample were combined. The extracts are then dried in a nitrogen evaporator in a water bath at room temperature. At this point, the samples were capped and frozen overnight.

The dried extracts were then thawed and reconstituted with 70 $\mu$L SDS/EDTA solution (0.11% sodium dodecyl sulfate (SDS), 15 mM EDTA, 0.9% NaCl) and 5 $\mu$L freshly prepared 1 mM DTE. 50 $\mu$L of freshly prepared $NaBH_4$ was then added to each tube. The tubes were vortexed and incubated at room temperature for 10 minutes. After 10 minutes, the samples were frozen at -70 °C. Before the solutions were thawed, 20 $\mu$L 2M HCl was added. After the solutions were thawed, 800 $\mu$L 100 mM $NH_4HCO_3$ was added. The solutions are vortexed and 5 $\mu$L of 100 mM monobromodiamane in acetonitrile solution (mBBr) was added. The solutions were then incubated in the dark for 90 minutes at room temperature.

Excess mBBr and the DTE derivative were removed from the samples after incubation by extraction with 1.5 mL dichloromethane. The aqueous layer was placed on the HPLC. The lipoic acid was separated using a mobile phase that consisted of 30% acetonitrile, 1% acetic acid, adjusted to pH 3.95 with about 2M $NH_4OH$ and was pumped at a flow rate of 1.0 mL/min with an isocratic elution for 15 minutes per injection. This preparation assumes that the density of the extruded food is equal to 1 g/mL.

Blood was collected aseptically for complete blood count, and blood biochemistry analysis 2 weeks prior to start and again at 0, 28, 56, 84, 112, 140 and 168 days of the study. In addition, 15 ml of whole blood was collected for isolation of lymphocytes at day 0, 28 and 84 of the dietary intervention.

Heparinzed whole blood was layered onto a 50 ml Accuspin conical centrifuge tube (Sigma Chemical) and an equal volume of Phosphate buffered saline (PBS) was added. Samples were centrifuged at 700 g for 30 minutes without brake. The monocyte layer was harvested, transferred to a 15 ml conical centrifuge tube, resuspended in 1-3 ml of PB, and centrifuged as before (First wash). A second wash was performed as the first wash. Finally, cells were harvested and suspended in perchloric acid (10% w/v) and frozen at -70 °C until analysis.

Samples were transferred from -70 °C freezer into a cooler with dry ice in it. Vials were centrifuged at 12,000 rpm for 5 minutes in a refrigerated centrifuge. An aliquot of supernatant for glutathione (GSH) analysis was transferred to a conical test tube.

Derivatization of the acid soluble extracts was by the method of Reed and coworkers (Fariss et al) as modified by Jones (Jones et al)

Briefly, 150 $\mu$L extract or external standards were added into a 1.5 ml Eppendorf tube followed by addition of 20 $\mu\gamma$-Glu-Glu internal standard and 50 $\mu$L IAA added followed by mixing. The solution was adjusted to pH about 10 (purple color) by using KOH-KHCO$_3$ working solution. Solutions were incubated 1 hr. under room temperature in the dark. Sanger's reagent was added at the same volume as of the total volume and the solution was incubated overnight (20 hrs) in the dark at room temperature.

After incubation, the solution was centrifuged at 12000 rpm for 5 minutes with the supernatant transferred into another 1.5 ml eppendorf tube. 200 $\mu$L supernatant was added into an amber autovial, which had a 300 $\mu$L inlet, fix the top with a crimper for HPLC analysis.

Solvents and separation conditions were as described (Fariss, Jones). Levels of GSH and GSSG were quantified relative to authentic standards. Gamma-glutamyl-glutamate was used as an internal standard to assess derivatization efficiency.

Comparison of values for clinical chemistry, hematology and body weights vs baseline were analyzed by way of paired t-test on SAS for windows with significance set at P<0.05. Means of values at each measured time point were separated by a one-way ANOVA with significance set at P<0.05. The difference in GSH:GSSG between day 84 and baseline were analyzed between groups by way of SAS for windows in a one-way ANOVA with significance set at P<0.05.

Results

Concentrations of lipoic acid (ppm) in food as determined over 7 successive assays (0, 28, 56, 84, 112, 140, 168 days) were within the range of expected assay sensitivity and production parameters typically encountered at our facility (Table 2).

The food intake data were unremarkable. Most animals in all groups ingested more food at 6 months, on average, than at the beginning of the study. Body weight data were unremarkable except that some weight loss occurred initially in the 4500 ppm inclusion group but that change appeared to reverse by 6 months time. Body condition scores did not appear to be affected by this minor loss of weight.

The routine physical examinations did not reveal any evidence of nutrition related abnormalities or dl-alpha-lipoic acid toxicity. All animals in the study population remained normal during the entire course of the study. Occasional vomiting was observed in several animals during the course of the study; however, a trend was not observed that would lead one to the conclusion that the vomiting may be attributable to lipoic acid. One animal, in the highest inclusion group, was dropped from the study at day 21 for weight loss and leukocytosis. The leukocytosis in this animal had not resolved by the end of the study and is suspected to be attributable to some other disease process.

When serum biochemistry values for days 28, 56, 84, 112, 140, and 168 were compared with the initial values for the same group of dogs, several statistical differences were noted, however, none of these were considered biologically significant because these values were within or very near the laboratory reference range and consistent trends over months were noted. Comparisons between the controls and the other treatment groups at each time period also revealed several statistical differences, however, none of these were considered biologically significant because these values were within or very near the clinical laboratory reference ranges and no trends were present.

When the hematology values for days 28, 56, 84, 112, 140 and 168 were compared with the initial values for the same group of dogs, several statistical differences were noted; however, none of these were considered biologically significant because these values were within or very near the laboratory reference range and not trends were present. Comparison between the controls and the other treatment groups at each time period revealed several statistical differences; however, none of these were considered biologically significant because these values were within or very near the clinical laboratory reference ranges and no trends were present.

GSH:GSSG ratio

The change in GSH:GSSG ratio over 84 days of feeding displayed a significant overall effect of diet (P=0.024) with all supplemented groups having an increase in the ratio (Table 3). ANOVA revealed a significant difference, compared to the basal food, for the lowest and highest inclusions, however, the largest numerical increase was in the lowest inclusion level. That is to say, the changes in the GSH:GSSG ratios for the highest and lowest inclusion were significantly different from the change observed over this same time period in the basal food. Ratios for 4 points could not be determined at day 84 as no GSSG was detectable in any of these samples (1 control, 3 treatment groups). As such, the values for supplemented groups may have displayed even higher ratios of GSH:GSSG if the assay had been sensitive enough to detect the low levels of GSSG at day 84.

**TABLE 2**

| Inclusion Rate Standard Percent (ppm) | Average | Standard Deviation | Target |
|---|---|---|---|
| 0 ppm | 24 ppm | 17 | N/A |
| 150 ppm | 151 ppm | 13 | 101 |
| 1,500 ppm | 1471 ppm | 113 | 98 |
| 3,000 ppm | 2869 ppm | 250 | 96 |
| 4,500 ppm | 4176 ppm | 642 | 93 |

**TABLE 3**

| Change In Mean Ratio Of GSH:GSSG From Day 0 To Day 84 In Dogs Consuming DL-Alpha Lipoic Acid In An Extruded Food | | | |
|---|---|---|---|
| Inclusion | Difference in GSH:GSSG ratio - day 0 to day 84 Inclusion compared to baseline food | N | P value |
| 0 ppm | -9.2 +/- 26 | 5* | NA |
| 150 ppm | 70 +/- 20 | 6 | 0.003 |
| 1,500 ppm | 24 +/- 7 | 6 | 0.16 |
| 3,000 ppm | 10 +/- 4 | 4* | 0.46 |
| 4,500 ppm | 50 +/- 36 | 4* | 0.03 |
| *1 dog in the control and 4,500 ppm group had no detectable GSSG at day 84 while 2 dogs in the 3,000 ppm group had no detectable GSSG at day 84. | | | |

Further observations with respect to alpha-lipoic acid are applicable. Chronic feeding of alpha-lipoic acid in diet is safe and effective. It improves the reduced glutathione (GSH) to oxidized glutathione (GSSG) ratio. The chronic administration of alpha-lipoic acid in the diet can be for periods of one, two, three, four, five, or six months minimum up through a period of one, two, three, four, five years or even more including the lifetime of the animal. The alpha-lipoic acid functions without any special protection in the diet such as encapsulation and need not be present in the diet in a unit dosage form such as those used in pharmaceuticals, for example, tablet, pill, capsule and the like. The lipoic acid is provided in the diet in a minimum of about 25, 50, 75, or 100 ppm of diet. The uppermost range is just below its toxic level, all the way down to about 400, 300, or 200 ppm of diet. Generally, one does not go beyond about 6 or 7 mg/kg body weight of animal per day, more generally not above about 5. The alpha-lipoic acid improves antioxidant defense capabilities as well as improves the animal's ability to resist oxidative damage. All this is done with the proper quantities of other antioxidants present such as vitamin E and vitamin C. This demonstrates that the action of alpha-lipoic acid is beyond that of vitamin C and/or vitamin E.

EXAMPLE 2

**Materials and Methods:**

30 dogs were used to determine the effect of lipoic acid when compared to an AAFCO control food or a test food containing fish oil.

A differential gene expression profile was studied between an AAFCO control food, a food containing fish oil, and a food containing lipoic acid. At a minimum of 1.3 fold change, a list of 1212 genes was generated compared to the AAFCO control and 1896 genes compared to the food containing fish oil at d 30.

**RNA extraction:** Total RNAs were isolated from whole blood samples using the PAXgene RNA isolation kit. All measurements were done with the canine 2 Affymetrix genechips. For statistical analysis, all measurements were normalized with RMA. All analysis was preformed using Partek. An ANOVA t-test was performed for genes that are differentially expressed between the control and test foods were selected based on p-value cutoff 0.1, fold change > +/- 1.3.

**Gene Expression:** Expression of 1212 genes was found to be altered in dogs fed a test food containing 150 ppm lipoic acid when compared to dogs fed an AAFCO control food. In addition, expression of 1896 genes was found to be altered in dogs fed the test food containing lipoic acid compared to a test food containing fish oil. Table 7 shows the genes grouped by function and the direction of expression relative to those fed either the control food or a food containing fish oil.

**Metabolomics:** Plasma metabolites were analyzed and were compared as fold change relative to the control fed dogs.

**Biomarker measurements:** Serum cartilage markers were measured using ELISA based kits to determine concentrations of cartilage markers. Day 0 was used as a covariate in the analysis to adjust for baseline values.

**Results:** The addition of lipoic acid to a food resulted in a greater decrease in type II collagen C-propeptide and carboxy-terminal crosslinked telopeptide fragment of type II collagen than a similar food without lipoic acid. These two markers are known to increase in dogs with osteoarthritis. Additionally, dogs fed a food containing lipoic acid lost fat suggesting an increase in utilization of fat for energy (fat oxidation) and a decreased production of glucose for healthier weight maintenance. In other words, dogs fed lipoic acid utilized available glucose more efficiently and shifted their metabolism towards mobilization of fat for energy use. Additionally, the metabolite hydroxyproline was reduced in plasma suggesting reduced cartilage destruction as this metabolite almost exclusively originates from cartilage. Furthermore, changes in gene expression as determined from serum white blood cells support the evidence to suggest increased cartilage protection from increased synthesis of cartilage components and decreased expression of enzymes that degrade cartilage. Finally, gene expression changes suggest improved fat utilization by increasing PDK4, which inhibits the formation of pyruvate from glucose and shifts metabolism to shuttling acetyl-CoA for energy, and upregulation of glucose transporters. The dogs fed lipoic acid also appeared genomically leaner compared to dogs fed the control food.

The tables show the difference in fat and weight as well as cartilage markers relative to treatment with lipoic acid.

Table 4 illustrates the change in body composition of dogs fed a control diet compared with dogs fed a control plus fish oil and a control plus lipoic acid. As illustrated in Table 4, dogs fed a control plus lipoic acid exhibited greatest change in weight over a 90 day treatment period.

| Table 4. Change in body composition over 90 days. | | | | | | | | |
|---|---|---|---|---|---|---|---|---|
| Treatment | | | | Probability, P < | | | | |
| | Control | Fish Oil | Lipoic Acid | SE | Treatment | Fish Oil vs. Control | Fish Oil vs. Lipoic Acid | Control vs. Lipoic Acid |
| Total Weight, g | 14,400 | 14,500 | 13,800 | 414.0 | 0.17 | 0.79 | 0.08 | 0.14 |
| Change in weight, g | 44.4 | 160.4 | -637.5 | 414.56 | 0.14 | 0.78 | 0.07 | 0.11 |
| Total fat, g | 3245.6 | 3175.3 | 2788.3 | 281.01 | 0.23 | 0.80 | 0.18 | 0.11 |
| Change in fat, g | -61.2 | -66.6 | -587.2 | 313.79 | 0.18 | 0.99 | 0.11 | 0.11 |

Table 5 illustrates the change in pyruvate blood levels in of dogs fed a control diet compared with dogs fed a control plus fish oil and a control plus lipoic acid. As illustrated in Table 5, dogs fed a control plus lipoic acid exhibited greatest change in pyruvate blood levels over a 30 day treatment period.

| Table 5. Fold change in pyruvate measured in the blood in dogs at day 30 fed three foods[a] | | | | | |
|---|---|---|---|---|---|
| | | | | Probability, P < | |
| Metabolite | Control | Lipoic acid vs Control | Lipoic acid vs Fish oil | + lipoic vs Control | + lipoic vs upgrade |
| Pyruvate | 1.000 | 0.5379 | 0.6274 | 0.01 | 0.03 |
| a. Day 0 values used as a covariate in the analysis. | | | | | |

Table 6 illustrates the percentage of dogs classified as genomically fat after being fed a control diet compared with dogs fed a control plus fish oil and a control plus lipoic acid for 30 days. As illustrated in Table 6, dogs fed a control plus lipoic acid genomically did not resemble physically fat dogs after a 30 day treatment period.

| Table 6: Percentage of Animals Classifying Genomically Fat | | | | | |
|---|---|---|---|---|---|
| | | | | Probability, P < | |
| Measure % | Control | Fish Oil | Lipoic Acid | Lipoic Acid vs. Control | Lipoic Acid vs. Fish Oil |
| **Percentage of animals at d 30 that classified fat using genomic markers** | 30 | 10 | 0 | **0.08** | 0.33 |

**Table 7. Genes related to cartilage and energy metabolism altered by lipoic acid compared to the control or upgrade foods (upgrade contains fish oil)**

| Gene name | Probe | Fold change | lipoic acid vs. |
|---|---|---|---|
| **Related to cartilage** | | | |
| **Gene name** | **Probe** | **Fold change** | **lipoic acid vs.** |
| **metabolism** | | | |
| Prolyl hydroxylase alpha 1 | CfaAffx.22481.1.S1_at | 1.4 | control |
| Prolyl hydroxylase alpha 2 | Cfa.13303.2.S1_a_at | 1.3 | control |
| Facilitated glucose transporter 9 | Cfa.7132.1.A1_at | 1.4 | control |
| TIMP1 | Cfa.3680.1.S1_s_at | 1.3 | control |
| Chondroitan sulfate synthase 1 | CfaAffx.16537.1.S1_at | 1.4 | control |
| heparan sulfate N-deacety lase/N-sulfotransferase 2 | Cfa.11897.1.A1_at | 1.3 | control |
| 12-lipooxygenase | CfaAffx.25908.1.S1_s_at | -1.3 | control |
| chondroitan sulfate proteoglycan 2 (veriscan) | CfaAffX.13597.1.S1_s_at | 1.5 | control |
| Lysyl hydroxylase | Cfa.16732.1A1_at | 1.3 | fish oil |
| N-acetylgalactosaminyltransferase 1 | Cfa.12862.1.S1_at | 1.3 | fish oil |
| Chondroitan sulfate synthase 1 | CfaAffx.16537.1.S1_at | 1.3 | fish oil |
| Fibronectin 1 | Cfa.3707.2.S1_at | 1.4 | fish oil |
| chondroitan sulfate proteoglycan 2 (veriscan) | CfaAffx.13597.1.S1_s_at | 1.5 | fish oil |
| ADAMTS-2 | Cfa.6326.1.A1_x_at | -1.3 | fish oil |
| ADAMTS-10 | | -1.3 | fish oil |
| ADAMTS-16 | CfaAffx.16270.1.S1_at | -1.3 | fish oil |
| 12-lipooxygenase | CfaAffx.25908.1.S1_s_at | -1.3 | fish oil |
| MMP2 | CfaAffx.14851.1.S1_s_at | -1.3 | fish oil |
| MMP7 | CfaAffx.23201.1.S1_at | -1.3 | fish oil |
| Transforming growth factor beta receptor 1 | Cfa.13340.1.A1_at | 1.3 | fish oil |

(continued)

| Gene name | Probe | Fold change | lipoic acid vs. |
|---|---|---|---|
| **Related to cartilage** | | | |
| **Gene name** | **Probe** | **Fold change** | **lipoic acid vs.** |
| **metabolism** | | | |
| Facilitated glucose transporter 9 | Cfa.7132.1.A1_at | 1.4 | fish oil |
| **Related to energy metabolism** | | | |
| **Gene name** | **Probe** | **Fold change** | **lipoic acid vs.** |
| PDK4 | Cfa.2282.1.S1_at | 1.4 | control |
| | Cfa.19125.2.S1_at, | | |
| Hexokinase 3 | CfaAffx.25391.1.S1_s_at | 1.3 | control |
| 5' AMP alpha 1 | Cfa.9738.1.S1_s_at | 1.3 | control |
| 5' AMP beta 1 | CfaAffx.15678.1.S1_at | 1.3 | control |
| 5' AMP gamma 2 | Cfa.10276.2.S1_a_at, | 1.4 | control |
| Facilitated glucose transporter 1 | CfaAffx.4630.1.S1_s_at | 1.3 | control |
| Facilitated glucose transporter 6 | Cfa.6832.1.A1_at | 1.3 | control |
| Succinyl CoA ligase alpha | Cfa.16185.1.S1_at | 1.3 | control |
| PPAR gamma | CfaAffx.8402.1.S1_s_at | 1.3 | control |
| Fatty acid desaturase 1 | CfaAffx.24518.1.S1_at | 1.9 | control |
| cAMP responsice element modulator | Cfa.855.1.S1_at | 1.3 | fish oil |
| PDK4 | Cfa.2282.1.S1_at | 1.6 | fish oil |
| | Cfa.19125.2.S1_at, | | |
| Hexokinase 3 | CfaAffx.25391.S1_s_at | 1.3 | fish oil |
| 5' AMP alpha 1 | Cfa.9738.1.S1_s_at | 1.3 | fish oil |
| 5' AMP beta 1 | CfaAffx.15678.1.S1_at | 1.3 | fish oil |
| 5' AMP gamma 2 | Cfa.10276.2.S1_a_at, | 1.5 | fish oil |
| Facilitated glucose transporter 1 | CfaAffx.4630.1.S1_s_at | 1.3 | fish oil |
| Facilitated glucose transporter 6 | Cfa.6832.1.A1_at | 1.4 | fish oil |
| Succinyl CoA ligase alpha | Cfa.16185.1.S1_at | 1.3 | fish oil |
| Succinyl CoA ligase beta | Cfa.1485.1.S1_at | 1.3 | fish oil |
| PPAR gamma | CfaAffx.8402.1.S1_s_at | 1.3 | fish oil |
| SREBP-1 | Cfa.189.2.S1_s_at | -1.3 | fish oil |

Table 8 illustrates the ingredients in an illustrative pet food composition of the invention.

**Table 8. Ingredients used to make composition**

| | Ingredients |
|---|---|
| 1 | Wheat |
| 2 | Milo |
| 3 | Corn |

(continued)

|  | Ingredients |
|---|---|
| 4 | Ground Chicken |
| 5 | Corn Gluten Meal |
| 6 | Poultry Meal |
| 7 | Soy bean oil |
| 8 | Flaxseed |
| 9 | Rice Brewers |
| 10 | Soybean meal, 49% |
| 11 | Pal enhancer 1 |
| 12 | Beet pulp |
| 13 | Potassium Citrate |
| 14 | Fish oil |
| 15 | DL-methionine |
| 16 | L-lysine HCl |
| 17 | Salt |
| 18 | Calcium carbonate |
| 19 | Lipoic acid |
| 20 | Choline chloride |
| 21 | Vitamin premix |
| 22 | L-threonine |
| 23 | Vitamin E |
| 24 | L-tryptophan |
| 25 | Lipoic acid |
| 26 | Mineral premix |
| 27 | Preservative |

Table 9 illustrates Dual Energy X-Ray Absorptiometry (DEXA) measurement for dogs after being fed a control diet compared with dogs fed a pet food including lipoic acid for 90 days. The measurement of body fat using DEXA is more accurate than body weight for assessing health. A dog can have a lot of muscle, but be considered "over-weight" by many height/weight charts. The opposite can also be true - a dog can have a lot of fat and little muscle and be "over-fat" but not overweight.

| Table 9. Canine Nutrigenomics DEXA (Dual Energy X-Ray Absorptiometry) at day 90 with initial as covariate | | | | | | | |
|---|---|---|---|---|---|---|---|
|  | Treatment | | |  | Probability, P < | | |
|  | Control | Upgrade | Upgrade +lipoic | SE | Upgrade vs Control | Upgrade vs +lipoic | control vs +lipoic |
| Total weight, kg | 14.4 | 14.5 | 13.8 | 0.41 | 0.79 | 0.08 | 0.14 |
| Food intake, g | 303.1 | 306.4 | 269.9 | 25.963 | 0.90 | 0.17 | 0.21 |
| Body condition score | 3.3 | 3.2 | 3.2 | 0.198 | 0.62 | 0.99 | 0.62 |
| BMC, g | 474.1 | 477.61 | 464.5 | 6.981 | 0.62 | 0.07 | 0.18 |

(continued)

| Table 9. Canine Nutrigenomics DEXA (Dual Energy X-Ray Absorptiometry) at day 90 with initial as covariate | | | | | | | |
|---|---|---|---|---|---|---|---|
| | Treatment | | | | Probability, P < | | |
| | Control | Upgrade | Upgrade +lipoic | SE | Upgrade vs Control | Upgrade vs +lipoic | control vs +lipoic |
| BMD, g | 0.89 | 0.90 | 0.88 | 0.012 | 0.43 | 0.20 | 0.61 |
| lean, g | 10699 | 10820 | 10558 | 220.73 | 0.59 | 0.25 | 0.53 |
| % fat | 21.54 | 21.59 | 20.69 | 1.643 | 0.98 | 0.59 | 0.61 |
| Total fat, g | 3245.6 | 3175.3 | 2788.3 | 281.01 | 0.80 | 0.18 | 0.11 |
| Lean:fat ratio | 4.15 | 4.20 | 4.25 | 0.425 | 0.90 | 0.92 | 0.82 |
| BMC, % | 3.3 | 3.3 | 3.3 | 0.10 | 0.78 | 0.67 | 0.48 |
| % lean | 74.8 | 75.4 | 76.4 | 1.50 | 0.67 | 0.48 | 0.26 |
| *Day 0 used as a covariate<br>- Control refers to standard AAFCO dog food.<br>- Upgrade refers to a low fat, reduced calorie, high fiber pet food | | | | | | | |

Table 10 illustrates body composition at day 180 to determine treatment effect with initial covariate for dogs fed five different pet foods for 180 days.

| Table 10. Canine body composition at day 180 Adult treatment effect with initial covariate | | | | | | | | | | |
|---|---|---|---|---|---|---|---|---|---|---|
| | Treatment | | | | | | Probability, P < | | | |
| | #1 | #2 | #3 | #4 | #5 | SE | vs #1 | vs #2 | Vs #4 | vs #5 |
| BMC, g | 419.78 | 395.51 | 400.1 | 442.07 | 393.07 | 24.95 | **0.05** | NS | NS | **0.05** |
| BMD, g | 0.601 | 0.584 | 0.587 | 0.623 | 0.577 | 0.0185 | NS | NS | NS | **0.05** |
| lean | 7929 | 7631 | 7758 | 7982 | 7380 | 479.6 | NS | NS | NS | NS |
| % fat | 24.2 | 25.50 | 26.5 | 28.8 | 27.20 | 2.480 | NS | NS | NS | NS |
| Total fat | 2726 | 2843 | 3012 | 3361 | 2945 | 365.6 | NS | NS | NS | NS |
| Total weight, kg | 11.1 | 10.9 | 11.2 | 11.8 | 10.7 | 681.3 | NS | NS | NS | NS |
| Lean: fat ratio | 3.28 | 3.01 | 2.86 | 2.57 | 2.95 | 0.405 | NS | NS | NS | NS |
| % BMC | 3.85 | 3.73 | 3.67 | 3.78 | 3.74 | 0.101 | NS | NS | NS | NS |
| % lean | 72.9 | 72.3 | 71.1 | 68.0 | 70.6 | 2.60 | NS | NS | NS | NS |

Table 11 illustrates change in metabolite measured in blood samples for dogs after being fed a control diet compared with dogs fed a pet food including lipoic acid for 30 days. As illustrated in Table 11, dogs fed a control plus lipoic acid displayed decreased kidney markers after 30 days.

| Table 11. Fold change in metabolites measured in the blood in dogs at day 30 fed three foods[a] | | | | | |
|---|---|---|---|---|---|
| | | | | Probability, P < | |
| Metabolite | Control | Upgrade vs Control | Upgrade vs Fish oil | Upgrade vs Control | Upgrade vs fish oil |
| Pyruvate | 1.0 | 0.54 | 0.63 | **0.01** | **0.03** |
| Hydroxyproline | 1.0 | 0.54 | 0.90 | **0.01** | 0.22 |

(continued)

| Table 11. Fold change in metabolites measured in the blood in dogs at day 30 fed three foods[a] | | | | | |
|---|---|---|---|---|---|
| | | | | Probability, P < | |
| Metabolite | Control | Upgrade vs Control | Upgrade vs Fish oil | Upgrade vs Control | Upgrade vs fish oil |
| 3-Indoxylsulfuric acid | 1.0 | 0.49 | 0.85 | **0.10** | 0.78 |
| 1,5 anhydrosorbitol | 1.0 | 0.75 | 1.10 | **0.01** | 0.33 |
| Nervonic acid | 1.0 | 1.40 | 1.05 | **0.01** | 0.24 |
| Alpha-tocopherol | 1.0 | 1.30 | 0.96 | **0.01** | 0.88 |
| Coenzyme Q10 | 1.0 | 1.84 | 1.29 | **0.01** | **0.13** |
| Allantoin | 1.0 | 0.97 | 1.25 | 0.78 | **0.02** |
| Creatine | 1.0 | 0.78 | 1.50 | **0.06** | **0.20** |
| Taurine | 1.0 | 1.41 | 0.98 | **0.01** | 0.98 |
| [a]Day 0 values used as a covariate in the analysis. - Control refers to standard AAFCO dog food. - Upgrade refers to a low fat, reduced calorie, high fiber pet food | | | | | |

The invention is not to be limited in scope by the specific embodiments disclosed in the examples, which are intended as illustrations of a few aspects of the invention.

The discussion of those references cited herein is intended merely to summarize the assertions made therein. No admission is made that any such patents, patent applications, publications or references, or any portion thereof, is relevant prior art for the present invention and the right to challenge the accuracy and pertinence of such patents, patent applications, publications, and other references is specifically reserved.

**Claims**

1. A composition comprising 100 ppm to 200 ppm alpha-lipoic acid for use in inducing loss of body fat or increasing the percentage of muscle mass in the treatment of a weight-related disorder in a companion animal in need thereof, which comprises feeding the companion animal said composition, wherein the companion animal is a dog.

2. The composition for use according to claim 1, wherein the composition is to be administered for at least 15 days.

3. The composition for use according to claim 2, wherein the composition is to be administered for at least 30 days.

4. The composition for use according to claim 3, wherein the composition is to be administered for at least 45 days.

5. The composition for use according to claim 1, wherein the composition is to be administered daily.

**Patentansprüche**

1. Zusammensetzung, die 100 ppm bis 200 ppm alpha-Liponsäure umfasst, zur Verwendung beim Induzieren des Verlustes von Körperfett oder Erhöhen der Prozentzahl an Muskelmasse bei der Behandlung einer gewichtsbezogenen Störung in einem Haustier, das dieses benötigt, was Füttern der Zusammensetzung an das Haustier umfasst, wobei das Haustier ein Hund ist.

2. Zusammensetzung zur Verwendung nach Anspruch 1, wobei die Zusammensetzung für mindestens 15 Tage verabreicht werden soll.

3. Zusammensetzung zur Verwendung nach Anspruch 2, wobei die Zusammensetzung für mindestens 30 Tage verabreicht werden soll.

**4.** Zusammensetzung zur Verwendung nach Anspruch 3, wobei die Zusammensetzung für mindestens 45 Tage verabreicht werden soll.

**5.** Zusammensetzung zur Verwendung nach Anspruch 1, wobei die Zusammensetzung täglich verabreicht werden soll.


**Revendications**

**1.** Composition comprenant 100 ppm à 200 ppm d'acide alpha-lipoïque destinée à être utilisée pour induire une perte de graisse corporelle ou augmenter le pourcentage de masse musculaire dans le traitement d'un trouble lié au poids chez un animal de compagnie en ayant besoin, qui comprend l'alimentation de l'animal de compagnie avec ladite composition, dans laquelle l'animal de compagnie est un chien.

**2.** Composition pour une utilisation selon la revendication 1, dans laquelle la composition doit être administrée pendant au moins 15 jours.

**3.** Composition pour une utilisation selon la revendication 2, dans laquelle la composition doit être administrée pendant au moins 30 jours.

**4.** Composition pour une utilisation selon la revendication 3, dans laquelle la composition doit être administrée pendant au moins 45 jours.

**5.** Composition pour une utilisation selon la revendication 1, dans laquelle la composition doit être administrée quotidiennement.

**REFERENCES CITED IN THE DESCRIPTION**

**Patent documents cited in the description**

- WO 2007063095 A
- US 20030224061 A
- DE 19818563
- JP 2007308468 A
- JP 2006219467 A
- US 2002006907 A
- US 5621117 A
- US 5339771 A
- US 5419283 A

**Non-patent literature cited in the description**

- **KIM et al.** *Nature Medicine,* 2004, vol. 10 (7), 727-733
- Nutrient Requirements of Swine. Nat'l Academy Press, 1998
- Nutrient Requirements of Poultry. Nat'l Academy Press, 1994
- Nutrient Requirements of Horses. Nat'l Academy Press
- American Feed Control Officials. Inc., Official publication, 2004, 129-137
- American Feed Control Officials. Inc. Official Publication, 2003, 220